(19) Europäisches Patentamt

European Patent Office

Office européen des brevets

(11) EP 2 542 104 B1

(12) **EUROPEAN PATENT SPECIFICATION**

(45) Date of publication and mention
of the grant of the patent:
**25.10.2017 Bulletin 2017/43**

(21) Application number: **11707898.0**

(22) Date of filing: **02.03.2011**

(51) Int Cl.:
**A24F 47/00** *(2006.01)* **A61M 15/06** *(2006.01)*

(86) International application number:
**PCT/GB2011/000285**

(87) International publication number:
**WO 2011/107737 (09.09.2011 Gazette 2011/36)**

(54) **A SIMULATED CIGARETTE**

ZIGARETTENSIMULIERENDES GERÄT

DISPOSITIF DE FAUSSE CIGARETTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.03.2010 GB 201003552**

(43) Date of publication of application:
**09.01.2013 Bulletin 2013/02**

(73) Proprietor: **Kind Consumer Limited
London EC1R 5AR (GB)**

(72) Inventors:
• **HEARN, Alex
London SE1 3UW (GB)**
• **MCDERMENT, Iain
Royston, Herts SG8 6EH (GB)**

(74) Representative: **Boult Wade Tennant
Verulam Gardens
70 Gray's Inn Road
London WC1X 8BT (GB)**

(56) References cited:
**EP-A1- 2 112 178        DE-A1- 4 030 257
US-A- 3 721 240         US-A1- 2008 241 255
US-A1- 2009 151 717**

## Description

The present invention relates to a simulated cigarette.

In the field of cigarette replacements, there have been a number of proposals to create a simulated cigarette. Such a device has a number of advantages over traditional nicotine replacement therapies such as patches and gum in that they recreate a physical act of smoking which is psychologically important to a smoker, and are also able to deliver nicotine in a dose which more closely replicates the pharmacokinetic effects of a cigarette that persistent smokers desire. Thus, a smoker is able to obtain the "hit" that is familiar from a cigarette, rather than having to deal with the slow release from a patch or gum which does not produce such a hit which leads to unpredictable dosings and poor craving scores and cessation rates.

The present invention is particularly concerned with a simulated cigarette device comprising a housing; a pressurised reservoir of inhalable composition within the housing; an outlet for the inhalable composition from the reservoir and out of the housing; and an outlet valve controlling the flow of inhalable composition through the outlet. Such a simulated cigarette will now subsequently be referred to as being "of the kind described".

One problem with a simulated cigarette of the kind described is the issue of orientation. In particular, if the device is to be effective, it is important that it is able to dispense its dose in practically any orientation.

In a traditional metered dose inhaler, the device has a bend or neck which alleviates the problem of orientation as the pressurised canister can always be accommodated in a vertical configuration with its outlet lowermost when a user inhales. As a simulated cigarette is designed to resemble a real cigarette, it has a narrow cylindrical construction and cannot, therefore, accommodate such a bend or neck.

As far as we are aware there have been two attempts in the prior art to address this problem.

DE 4,030,257 discloses that the outlet valve is connected to the end of a flexible immersion tube. It is stated that the immersion tube, largely independent of the respective position of the device or sleeve, is always immersed with its open end in the fluid active ingredient present in the chamber. However, this immersion tube, which is presumably weighted, may help in some orientations, but will not allow continuous use in all orientations. For example, if the reservoir is less than half full, the user could inhale on the device but receive no aerosolised dose if the cigarette is in a 'tip down' orientation.

The second document is US 3,721,240. This discloses a simulated cigarette device with a pressurised reservoir and an outlet valve at one end. The outlet valve is associated with an evaporation strip or wick. This is shown as a thin strip extending from the outlet valve directly to the lower surface of the reservoir and would appear to extend less than half of the length of the reservoir.

Thus, although the wick will transfer liquid has a accumulated at the bottom of the reservoir in the region adjacent to the outlet valve, it seems incapable of having any effect if, say, the reservoir is less than half full and is used in a 'tip-down' orientation. Further, the wick would only seem to operate if the simulate cigarette device is in the correct angular orientation (i.e. in the correct orientation along its main longitudinal axis). If the simulated cigarette were to be used upside down to the illustrated configuration, if the wick is attached to the wall of the reservoir, it will have no effect at all. If it is not attached to the wall, it would simply droop down into the reservoir at a location immediately adjacent to the outlet valve and therefore would have little or no effect. Thus, the wick disclosed in US 3,721,240 may be fully operational only if the simulated cigarette is used in the correct angular orientation and, even then, would be of limited benefit.

A further simulated cigarette device which is less relevant than US 3,721,240 but does bear some superficial similarities with the present invention is that disclosed in FR 2 650 002. This does not disclose a pressurised reservoir. Instead, it discloses a porous matrix which acts to retain the inhalable volatile composition. The porous matrix is contained between a pair of flap valves. When a user sucks on the device, the suction force opens the two flap valves allowing air to be drawn through the porous matrix entraining some of the volatile composition. The porous matrix is simply designed to retain the substance and provides no wicking function whatsoever.

The present invention aims, for the first time, to provide a simulated cigarette device of the kind described which can be used in practically all orientations.

According to the present invention, a simulated cigarette device of the kind described is characterised by a capillary plug extending from the vicinity of the outlet valve into the reservoir, filling at least 50% of the reservoir and being configured to wick the inhalable composition towards the outlet valve.

By using the capillary plug which fills a substantial portion of the reservoir, rather than the small thin wick of US 3,721,240, the present invention provides a simulated cigarette which, for the first time, can be used in practically any orientation. As the plug fills a substantial portion of the reservoir, the orientation of a cigarette is far less significant as it will tend to absorb the liquid to a greater or lesser extent in any orientation. Further, by filling a substantial portion of the reservoir, the capillary plug is not confined to the region adjacent to the outlet valve so that it can absorb liquid which is some distance from the outlet valve and transfer this to the outlet valve.

In order to fill a substantial portion of the reservoir, the plug preferably occupies at least 60% and more preferably at least 70% of the volume of the reservoir. Preferably, the plug extends at least 70% and, more preferably, at least 80% of the length of the reservoir.

On the other hand, it is preferable that the plug does not fill the entire reservoir. This is because the space occupied by the wicking material reduces the capacity of the reservoir. Therefore, preferably, at least 10%, but more preferably at least 20% of the volume of the reservoir is not occupied by the wicking material.

The simulated cigarette preferably has a refill valve at the end opposite to the outlet end. Preferably, there is a gap between the refill valve and the end of the plug to allow an unobstructed space for the refill liquid to enter the reservoir. Preferably, also, the plug does not occupy the full cross-section of the reservoir for a substantial proportion of the length of the reservoir to allow a liquid flow path within the reservoir alongside the porous plug. This ensures that some of the refill liquid can travel along this path before being absorbed laterally into the porous plug. This allows the wick to be saturated more readily than it would be if the plug filled the entire cross-section of the reservoir such that all of the refill liquid needed to flow along the porous plug.

Preferably, the capillary uptake of the plug is such that the composition at the distal end of the plug is drawn towards the outlet valve for a distance of at least 25mm, and more preferably at least 30mm when the simulated cigarette is held in a position with the outlet end uppermost. Thus, this requires a material which provides strong capillary action.

In order to enhance the capillary action, the plug preferably has a pore size which decreases towards the outlet valve. This may be achieved by using a graduated material which may be a single phase material formed with varying pore size, or may be two or more materials each having different pore sizes, the relative proportions of which are varied along the length of the plug. However, preferably, this is achieved by compressing the plug in the vicinity of the outlet valve. This provides a simple means of achieving the variable pore size and also is readily achievable in a design where the opening/closing mechanism for the outlet valve lies alongside the portion of the reservoir at the end adjacent to the outlet valve as the reservoir is naturally thinner at this point.

In order to select appropriate foam wicking characteristics, two useful parameters describing foam wicking performance are the maximum rise-height for a given fluid, which describes the maximum height a fluid can rise up in the foam due to capillary forces when the foam is dipped into a pool of the fluid and is inversely proportional to the size of pores in the foam; and the foam permeability (which is fluid-independent), which describes the speed at which fluid can flow through the foam in response to a pressure gradient, and is proportional to the pore area, and the empty volume fraction of the foam. In practice, the time taken to approach the maximum rise-height and the permeability can be optimally selected to leave little residual liquid in the reservoir.

When detailing the precise characteristics, the pore radii are between 50 and 500 microns, preferably 100-300 and most preferably 100-150 microns. The capillary plug needs to be hydrophilic in order to provide the hydrostatic arrangement to the outlet valve, and this is helped by the small pore size, especially in situ at the outlet valve. This can be best achieved with a cross-lined open cell, reticulated structure. In addition, it is envisaged that the compression of foam at the outlet valve should entertain pore radii most preferably around 100 microns, whereas the base of the wicking apparatus should constitute pore sizes of around 150 microns to provide an optimum capillary gradient.

Preferably, the density of the plug is between 1% and 50%, preferably between 1% and 10%m, and most preferably between 5% and 6% (defined as the ratio of the volume of the solid material and the volume of the voids). For a 6% density, this means that the fill of the chamber is not jeopardised by the volume of foam incorporated, and so does not limit the number of inhalations a user will derive from the device. Medium and High-Density capillary rods are 60 $(kg/m^3)$ and 120 $(kg/m^3)$, where the greater the density the higher the rise height of liquid through the capillary rod. The majority of the liquid containing solution will be of a liquid propellant e.g. HFA134b but can also be used with any other liquid propellant or liquidified gases e.g carbon dioxide or nitrogen. Using a surface tension of 8.69 $e^{-3}$ n/m for the HFA134b, and a density of 1220 $kg/m^3$, testing results obtained from the device give approximately 5.4mm rise height with a medium density Capu-Cell foam, and approximately 6.7mm rise height with a high density Capu-Cell. Since the density of the propellant based solution differs from a water based solution a number of small modifications have to take place in order to increase rise height - for example the density of the foam needs to be increased and the pore radii decreases by approximately 5-10%. The aim is to ensure that the rise-height is sufficient to travel the full length of the capillary plug and engage the outlet valve, even if there is only a small amount of remaining fluid in the receptacle.

The pore radii and the density of the foam should be kept in equilibrium as to provide the maximum amount of performance for the device. A capillary plug which has cells that are too small might ultimately limit the flowrate of formulation from the device and so the performance of the foam must ensure that the delivery of the flow from any angle of orientation is between 60 and 80mm$^3$ per second, in order to ensure accuracy and consistency in the emitted dose.

$$h_{\max} = \frac{2\gamma}{\rho g R_{pore}}$$

$g$ = gravitational acceleration, $\gamma$ = fluid surface tension,

$\rho$ = fluid density and $R_{pore}$ = foam pore-size

For solutions where the fluid density and surface tension are different - i.e. by changing the combinations of propellant (for example the more dense HFA227), excipient or active ingredient, the capillary plug can have a pore size and density level, which can be graduated across the device, to fit the optimum performance of the flow of aerosol for the user. With this in mind, not only can pore size be graduated upwards towards the distal end, pore size can be graduated across the device, so that smaller pore sizes can form the core of the wicking area, and surround the outlet valve. In practical terms, this is achieved by layering smaller pore sized foam sheet onto larger sheets so that there is a graduation in pore size over the cross sectional area. This means than pore size can graduate across the device as well as along the device to increase performance. In the present case, the plug has been calibrated with the use of a nicotine containing solution.

The foam permeability (which is fluid independent), describes the speed at which fluid can flow through the foam in response to a pressure gradient, according to the equation where k is permeability, $\epsilon$ is the the empty volume fraction of the foam, $\tau$ is known as "tortuosity" and is a way of taking account of the twists and turns that the fluid must take to pass through the foam, and $R^2$ (pore) equates to pore size:

$$k \approx \frac{\epsilon}{\tau} \frac{R_{pore}^2}{8}$$

The permeability ($m^2$) of the wicking apparatus is preferably between $e^{-3}$ and $e^{-25}$, more preferably $e^{-10}$ and $e^{-15}$, and most preferably between $e^{-10}$ and $e^{-12}$. A functional example of this is a selected polyolefin wick material which has a permeability of $1.87e^{-11}$ $m^2$.

However other preferred materials for a plug are polyurethane, Capu-Cell, a polyether/polyester hybrid, neoprene, Basotect and PVA. These materials have sufficient capacity to absorb the inhalation composition which can contain a propellant HFA 134a, HFA 227, CFC, Freon or any other propellant or gas in liquid phase form. It is a preferred embodiment that neoprene, PU or Basotect is used, since all have similar rise heights when tuned for pore radii and density, where the pore radii can be compressed or modified to suit. Preferably the wicking material demonstrates good chemical resistance, and most preferably is medically compatible e.g. Scapa medical foams, absorbent polyurethane foams designed for wound cleaning, Intec hydrophilic and reticulated foams or rubber foams, and display good compatibility with nicotine as regarding degradation, extractables and leechables.

Examples of simulated cigarettes in accordance with the present invention will now be described with reference to the accompany drawings, in which:

Fig. 1 is an exploded perspective view of the simulated cigarette device;
Fig. 2 is a cross-section of the device of Fig. 1; and

Figs. 3 to 8 are cross-sectional perspective diagrams showing several wicking materials of different shapes, densities and capillary rate uptakes.

The present invention relates to a capillary rod with hydrostatic enabling of the outlet valve for a breath-activated cigarette and only this aspect of the invention will be specifically described here. For details of the construction of the remainder of the cigarette device and its refill mechanism, reference is made to WO 2009/001078.

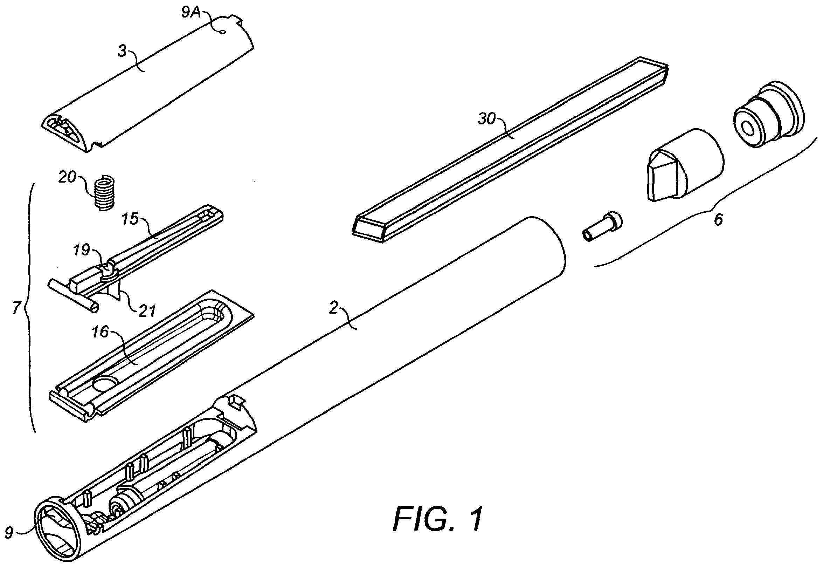

The device has a housing 1 made up of a main chassis 2 and a closure element 3 as shown in Fig. 1. This is held in place by label (not shown). Within the housing, there is a pressurised reservoir 5 containing the inhalable composition. It may be refillable as described in WO 2009/001082 through the filling valve 6, or the device may be a single use device, or may be arranged so that the reservoir 5 is a replaceable component.

The breath-activated valve 7 is positioned between an outlet end 8 and the reservoir 5. The breath-activated valve is arranged so that, when a user sucks on the outlet end 8, the breath-activated valve 7 opens to allow the inhalable composition from the reservoir 5 to be inhaled.

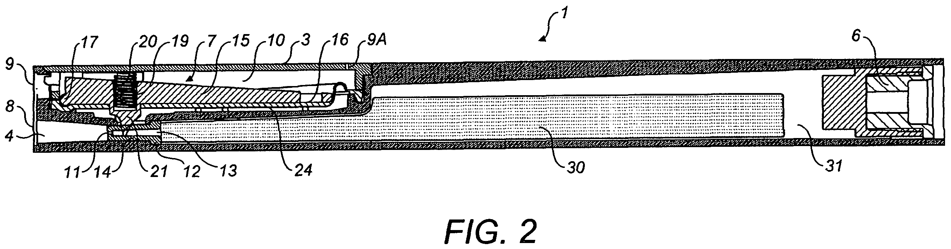

The housing at the outlet end has two orifices. The first of these is the suction orifice 9 which communicates with a chamber 10 as will be described in greater detail below and the second is an outlet orifice 11 from which the inhalable composition dispensed is also described in more detail below. As is apparent from Fig. 2, the outlet orifice 11 is provided on a separate component 12.

An outlet path 13 is defined between the reservoir 5 and outlet orifice 11 provided by deformable tubular element 14. This tubular element is moved between closed and open positions shown by a mechanism which will now be described.

This mechanism comprises a pivotally mounted vane 15 and a membrane 16. The pivotally mounted vane has a pivot 17 at the end closest to the outlet end 8 and a central reinforcing rib 18 running along its length and tapering away from the outlet end. At around the midpoint, the vane 15 is provided with a recess 19 for receiving a spring 20 which biases it into the closed position shown in Fig. 1 (although the deformable tubular element is shown in its undeformed state). Below the recess 19 is a jaw 21 having a triangular cross-section which is configured to apply the force provided from the vane 15 to the deformable tube 14 over a narrow area. The vane 15 is supported by the diaphragm 16 which is sealed to the housing at its ends 22, 23. This seals off the chamber 10 other than to the suction orifice 9 and an air vent that is located in the closure element 3.

The underside 24 of the membrane 16 is open to atmospheric pressure as a leakage path exists through the housing 1 which is not shown in the drawings as it extends around the outlet path 1 and is therefore not shown in the plane of Fig. 1.

When a user sucks on the outlet end 8, the suction is communicated by the suction orifice 9 to the chamber 10 thereby lowering the pressure in this chamber. This causes the vane 15 to be lifted against the action of the spring 20 deforming the diaphragm and lifting the jaw 21 to allow the deformable tube to open, thereby allowing the inhalable composition from the reservoir 5 along outlet path 13 through the deformable tube 14 and out through the outlet orifice 11. The degree of suction applied by the user will determine the extent to which the vane 15 moves and therefore the amount of composition that the user receives. As soon as a user stops sucking, atmospheric pressure will return to the chamber 10 via the suction orifice 9 and the spring 20 will return the vane thereby pinching the tube 14 closed.

Within the reservoir is a capillary plug 30. As is apparent from Fig. 2, this extends for substantially the entire length of the reservoir, although there is a gap 51 between the end of the rod and the refill valve 6. As can also be seen in Fig. 2, the plug 30 does not fill the entire cross-section of the reservoir, at least in the region away from the valve 7. Instead, a gap 32 exists along the top surface of the rod. This allows refill material to pass along this gap and be absorbed along the length of the rod, rather than all of the refill liquid having to enter through the end of the rod.

In the region adjacent to the valve 7, the reservoir is significantly thinner. In this region, the rod 30 is compressed thereby reducing the pore size to provide increased capillary force in this region.

It will be appreciated from this that, even when the reservoir is almost empty, whichever orientation it is in, the liquid will come into contact with the capillary rod 30. From there, the rod is readily able to wick the liquid to the outlet path 13.

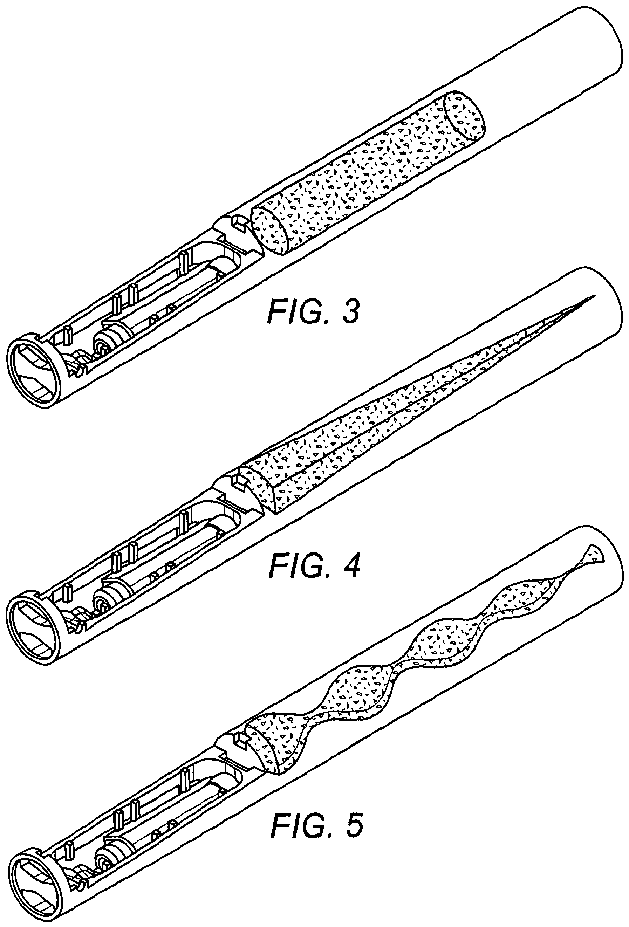

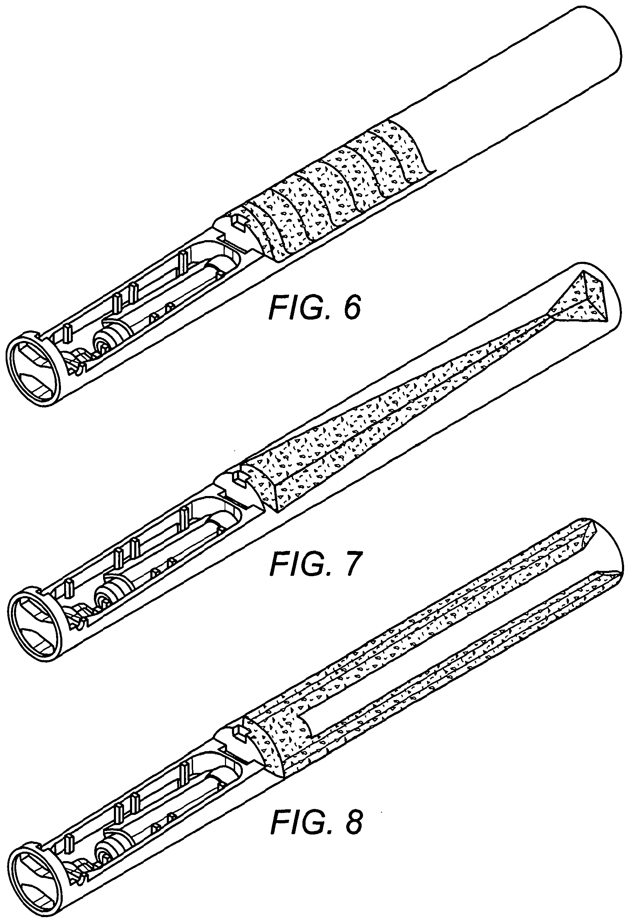

A number of alternative wick arrangements are shown in Figs. 3 to 8. All of these have in common that they fill a substantial portion of the reservoir and reach a significant distance from the outlet valve to the opposite end.

## Claims

1. A simulated cigarette device comprising a housing (1); a pressurised reservoir (5) of inhalable composition within the housing; an outlet (11) for the inhalable composition from the reservoir and out of the housing; an outlet valve (7) controlling the flow of inhalable composition through the outlet; and a capillary plug (30) extending from the vicinity of the outlet valve into the reservoir, **characterised by** the capillary plug (30) filling at least 50% of the reservoir and being configured to wick the inhalable composition towards the outlet valve.

2. A device according to claim 1, wherein the plug (30) occupies at least 60% and more preferably at least 70% of the volume of the reservoir (5).

3. A device according to claim 1 or claim 2, wherein the plug (30) extends for at least 70% and more preferably at least 80% of the length of the reservoir (5).

4. A device according to any one of the preceding claims, wherein at least 10%, more preferably at least 20% of the volume of the reservoir (5) is not occupied by the wicking material.

5. A device according to any one of the preceding claims, further comprising and a refill valve (6) at the end opposite to the outlet (11) and a gap between the refill valve and the end of the plug (30) to allow an unobstructed space for the refill liquid to enter the reservoir.

6. A device according to any one of the preceding claims, wherein the plug (30) does not occupy the full cross-section of the reservoir (5) for a substantial proportion of the length of the reservoir to allow a liquid flow path within the reservoir alongside the porous plug.

7. A device according to any one of the preceding claims, wherein the capillary uptake of the plug (30) is such that the composition at the distal end of the plug is drawn towards the outlet valve (7) for a distance of at least 25mm, and more preferably at least 30mm when the simulated cigarette is held in a position with the outlet end uppermost.

8. A device according to any one of the preceding claims, wherein the plug (30) has a pore size which decreases towards the outlet valve.

9. A device according to claim 8, wherein the decreasing pore size is achieved by compressing the plug (30) in the vicinity of the outlet valve.

10. A device according to any one of the preceding claims, wherein in a transverse plane across the plug (30), the pore size is smaller in the central region and larger at the peripheral region.

11. A device according to any one of the preceding claims, wherein the pore radii are between 100 and 500 microns, preferably 100-300 and most preferably 100-150 microns.

12. A device according to any one of the preceding claims, wherein the permeability ($m^2$) of the plug (30) is preferably between $e^{-3}$ and $e^{-25}$, more preferably $e^{-10}$ and $e^{-15}$, and most preferably between $e^{-10}$ and $e^{-12}$.

## Patentansprüche

1. Zigarettensimulierendes Gerät, aufweisend ein Gehäuse (1); ein druckbeaufschlagtes Reservoir (5) inhalierbarer Zusammensetzung in dem Gehäuse; einen Auslass (11) aus dem Reservoir und aus dem Gehäuse für die inhalierbare Zusammensetzung; ein Auslassventil (7), das den Fluss an inhalierbarer Zusammensetzung durch den Auslass kontrolliert; und einen Kapillarpfropf (30), der sich von der Nachbarschaft des Auslassventils aus in das Reservoir hinein erstreckt, **dadurch gekennzeichnet, dass** der Kapillarpfropf (30) mindestens 50% des Reservoirs ausfüllt und dazu ausgelegt ist, die inhalierbare Zusammensetzung in Richtung des Auslassventils zu transportieren.

2. Gerät nach Anspruch I, wobei der Pfropfen (30) mindestens 60% und stärker bevorzugt mindestens 70% des Volumens des Reservoirs (5) einnimmt.

3. Gerät nach Anspruch 1 oder Anspruch 2, wobei sich der Pfropfen (30) auf mindestens 70% und stärker bevorzugt mindestens 80% der Länge des Reservoirs (5) erstreckt.

4. Gerät nach einem der vorangehenden Ansprüche, wobei mindestens 10%, stärker bevorzugt mindestens 20% des Volumens des Reservoirs (5) nicht von dem Dochtmaterial eingenommen werden.

5. Gerät nach einem der vorangehenden Ansprüche, weiterhin aufweisend ein Nachfüllventil (6) am Ende gegenüber dem Auslass (11) und einen Spalt zwischen dem Nachfüllventil und dem Ende des Pfropfens (30), um einen unversperrten Raum zum Eintritt der Nachfüllflüssigkeit in das Reservoir zu ermöglichen.

6. Gerät nach einem der vorangehenden Ansprüche, wobei der Pfropfen (30) nicht den vollen Querschnitt des Reservoirs (5) auf einem wesentlichen Abschnitt der Länge des Reservoirs einnimmt, um einen Flüssigkeitsströmungsweg in dem Reservoir entlang des porösen Pfropfens zu ermöglichen.

7. Gerät nach einem der vorangehenden Ansprüche, wobei die kapillare Aufnahme des Pfropfens (30) so ist, dass die Zusammensetzung am distalen Ende des Pfropfens auf einer Distanz von mindestens 25mm, und stärker bevorzugt mindestens 30mm in Richtung des Auslassventils (7) gezogen wird, wenn die simulierte Zigarette in einer Position mit dem Auslassende zuoberst gehalten wird.

8. Gerät nach einem der vorangehenden Ansprüche, wobei der Pfropfen (30) eine Porengröße aufweist, die in Richtung des Auslassventils abnimmt.

9. Gerät nach Anspruch 8, wobei die abnehmende Porengröße durch Kompression des Pfropfens (30) in der Nachbarschaft des Auslassventils erreicht wird.

10. Gerät nach einem der vorangehenden Ansprüche, wobei in einer Querebene über den Pfropfen (30) hinweg, die Porengröße im Mittelbereich kleiner und im peripheren Bereich größer ist.

11. Gerät nach einem der vorangehenden Ansprüche, wobei die Porenradien zwischen 100 und 500 $\mu$m, vorzugsweise 100-300 $\mu$m und besonders bevorzugt 100-150 $\mu$m betragen.

12. Gerät nach einem der vorangehenden Ansprüche, wobei die Permeabilität (m$^2$) des Pfropfens (30) vorzugsweise zwischen e$^{-3}$ und e$^{-25}$, stärker bevorzugt zwischen e$^{-10}$ und e$^{-15}$ und besonders bevorzugt zwischen e$^{-10}$ und e$^{-12}$ beträgt.

## Revendications

1. Dispositif de fausse cigarette comprenant un boîtier (1) ; un réservoir sous pression (5) de composition inhalable dans le boîtier ; une sortie (11) pour la composition inhalable à partir du réservoir et hors du boîtier ; une soupape de sortie (7) régulant l'écoulement de composition inhalable à travers la sortie ; et un bouchon capillaire (30) s'étendant à partir du voisinage de la soupape de sortie dans le réservoir, **caractérisé en ce que** le bouchon capillaire (30) remplit au moins 50 % du réservoir et est configuré pour acheminer par effet de mèche la composition inhalable vers la soupape de sortie.

2. Dispositif selon la revendication 1, dans lequel le bouchon (30) occupe au moins 60 % et plus préférablement au moins 70 % du volume du réservoir (5).

3. Dispositif selon la revendication 1 ou 2, dans lequel le bouchon (30) s'étend sur au moins 70 % et plus préférablement au moins 80 % de la longueur du réservoir (5).

4. Dispositif selon l'une quelconque des revendications précédentes, dans lequel au moins 10 %, plus préférablement au moins 20 % du volume du réservoir (5) n'est pas occupé par le matériau à effet de mèche.

5. Dispositif selon l'une quelconque des revendications précédentes, comprenant en outre une soupape de remplissage (6) au niveau de l'extrémité opposée à la sortie (11) et un interstice entre la soupape de remplissage et l'extrémité du bouchon (30) pour permettre d'obtenir un espace dégagé pour que le liquide de remplissage entre dans le réservoir.

**6.** Dispositif selon l'une quelconque des revendications précédentes, dans lequel le bouchon (30) n'occupe pas la totalité de la section transversale du réservoir (5) pour une proportion importante de la longueur du réservoir pour permettre d'obtenir un trajet d'écoulement de liquide dans le réservoir le long du bouchon poreux.

**7.** Dispositif selon l'une quelconque des revendications précédentes, dans lequel l'absorption capillaire du bouchon (30) est telle que la composition au niveau de l'extrémité distale du bouchon est aspirée vers la soupape de sortie (7) sur une distance d'au moins 25 mm, et plus préférablement d'au moins 30 mm lorsque la fausse cigarette est maintenue dans une position avec l'extrémité de sortie étant la plus haute.

**8.** Dispositif selon l'une quelconque des revendications précédentes, dans lequel le bouchon (30) a une taille des pores qui diminue vers la soupape de sortie.

**9.** Dispositif selon la revendication 8, dans lequel la taille des pores décroissante est obtenue en comprimant le bouchon (30) au voisinage de la soupape de sortie.

**10.** Dispositif selon l'une quelconque des revendications précédentes, dans lequel, dans un plan transversal coupant le bouchon (30), la taille des pores est plus petite dans la région centrale et plus grande au niveau de la région périphérique.

**11.** Dispositif selon l'une quelconque des revendications précédentes, dans lequel les rayons des pores sont compris entre 100 et 500 microns, de préférence entre 100 et 300 et idéalement entre 100 et 150 microns.

**12.** Dispositif selon l'une quelconque des revendications précédentes, dans lequel la perméabilité ($m^2$) du bouchon (30) est de préférence comprise entre $e^{-3}$ et $e^{-25}$, plus préférablement entre $e^{-10}$ et $e^{-15}$, et idéalement entre $e^{-10}$ et $e^{-12}$.

FIG. 1

FIG. 2

EP 2 542 104 B1

*FIG. 3*

*FIG. 4*

*FIG. 5*

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4030257
- US 3721240 A
- FR 2650002
- WO 2009001078 A
- WO 2009001082 A